# EUROPEAN PATENT SPECIFICATION

(11) **EP 3 360 553 B1**
(45) Date of publication and mention of the grant of the patent: **03.03.2021**
(21) Application number: 17305149.1
(22) Date of filing: 09.02.2017
(51) Int. Cl.: A61K 31/428, A61P 7/00

(54) **MOLECULES AND PHARMACEUTICAL COMPOSITIONS, IN PARTICULAR FOR TREATING A GENETIC BLOOD DISORDER**
MOLEKÜLE UND PHARMAZEUTISCHE ZUSAMMENSETZUNGEN, INSBESONDERE ZUR BEHANDLUNG EINER GENETISCHEN BLUTKRANKHEIT
MOLÉCULES ET COMPOSITIONS PHARMACEUTIQUES, EN PARTICULIER POUR LE TRAITEMENT D'UN TROUBLE SANGUIN GÉNÉTIQUE

(43) Date of publication of application: 15.08.2018
(73) Proprietor: Université de La Réunion, 97400 Saint Denis La Réunion (FR)
(72) Inventor: GARDEBIEN, Fabrice, 97715 LA RÉUNION (FR); MADELEINE, Noëlly, 97715 LA RÉUNION (FR); EL NEMER, Wassim, 75739 PARIS CEDEX 15 (FR); COCHET, Sylvie, 75739 PARIS CEDEX 15 (FR)
(74) Representative: Lavoix

(56) References cited:
- CN-A- 105 440 085
- US-A- 4 696 937
- DATABASE REGISTRY [Online] CHEMICAL ABSTRACTS SERVICE, COLUMBUS, OHIO, US; 28 February 2003 (2003-02-28), XP002772103, retrieved from STN accession no. 496048-54-5 Database accession no. 496048-54-5
- DATABASE REGISTRY [Online] CHEMICAL ABSTRACTS SERVICE, COLUMBUS, OHIO, US; 6 April 2010 (2010-04-06), XP002772104, retrieved from STN accession no. 1217231-78-1 Database accession no. 1217231-78-1
- KATAVA ROBERT ET AL: "Supramolecular solid-state structure, potential energy surfaces and evaluation of antiproliferative effect of 2-benzothiazolylhydrazone derivatives in vitro", STRUCTURAL CHEMISTRY, DEERFIELD BEACH, FL, US, vol. 28, no. 3, 1 October 2016 (2016-10-01), pages 709-721, XP036226751, ISSN: 1040-0400, DOI: 10.1007/S11224-016-0856-0 [retrieved on 2016-10-01]
- M A SAYYED: "Synthesis and antibacterial activity of some new benzylidenes-2-hydrazino benzothiazole", INT. J. CHEM. SCI., vol. 1, no. 4, 1 January 2003 (2003-01-01) , pages 407-409, XP055390205,
- MANKELOW TOSTI J ET AL: "The Laminin 511/521-binding site on the Lutheran blood group glycoprotein islocated at the flexible junction of Ig domains 2 and 3", B, THE AMERICAN SOCIETY OF HEMATOLOGY, US, vol. 110, no. 9, 1 November 2007 (2007-11-01), pages 3398-3406, XP008090091, ISSN: 0006-4971, DOI: 10.1182/BLOOD-2007-06-094748

## Description

The present invention relates to molecules and pharmaceutical compositions. More precisely, The present invention relates to molecules and pharmaceutical compositions for treating a genetic blood disorder, and in particular drepanocystosis.

Drepanocystosis or sickle-cell disease (SCD), is a group of genetic blood disorders characterized by red blood cells that assume an upnormal, rigid, sickle shape. The most common type is known as sickle-cell anaemia (SCA). It results in an abnormality in the oxygen-carrying protein haemoglobin found in red blood cells. Sickle-cell disease occurs when a person inherits from its parents two abnormal copies of haemoglobin gene. More than 3 million people have sickle-cell disease and more than 40 million have sickle-cell trait, i.e. persons with a single abnormal gene copy which does not usually have symptoms of the disease. Sickle-cell disease could lead to acute and chronic complications, including those with a high mortality rate.

In the pathogenesis of vaso-occlusive crisis of sickle cell disease, red blood cells bind to the endothelium and promote vaso-occlusion. At the surface of these sickle red blood cells, the overexpressed protein Lutheran strongly interacts with the Laminin 511/521. Accession number in Uniprot (Uniprot.org) for Lutheran (Lu) : P50895 (http://www.uniprot.org/uniprot/P50895) . Alternative name for Lu are: Auberger B antigen; B-CAM cell surface glycoprotein; F8/G253 antigen; Lutheran antigen; Lutheran blood group glycoprotein; CD_antigen: CD239. The main references on Lu are:
"The Lutheran blood group glycoprotein, another member of the immunoglobulin superfamily, is widely expressed in human tissues and is developmentally regulated in human liver." Parsons S.F., Mallinson G., Holmes C.H., Houlihan J.M., Simpson K.L., Mawby W.J., Spurr N.K., Warne D., Barclay A.N., Anstee D.J. Proc. Natl. Acad. Sci. U.S.A. 92:5496-5500(1995);
"Basal cell adhesion molecule/lutheran protein. The receptor critical for sickle cell adhesion to laminin." Udani M., Zen Q., Cottman M., Leonard N., Jefferson S., Daymont C., Truskey G., Telen M.J. J. Clin. Invest. 101:2550-2558(1998);
"The Laminin 511/521-binding site on the Lutheran blood group glycoprotein is located at the flexible junction of Ig domains 2 and 3." Mankelow T.J., Burton N., Stefansdottir F.O., Spring F.A., Parsons S.F., Pedersen J.S., Oliveira C.L., Lammie D., Wess T., Mohandas N., Chasis J.A., Brady R.L., Anstee D.J. Blood 110:3398-3406(2007).

Sickling decreases the cells' flexibility and results in a risk of various complications. The hallmark of sickle cell disease is episodic and painful vaso-occlusion. Vaso-occlusion is caused by a strong adhesion of sickle red blood cells and leukocytes to the vascular endothelium by adhering to the vascular endothelium, erythrocytes and leukocytes reduce the lumen of the vessels and reduce the blood flow. In vitro studies indicate that the adhesion of sickle erythrocytes to the vascular endothelium is important in the pathogenesis of vaso-occlusive crisis of sickle cell disease. This phenomenon is explained by an abnormal expression and activation of several membrane proteins on the surface of sickle red blood cells that strongly interact with the components of the endothelium and the sub-endothelial matrix. Adhesion proteins which are responsible for vaso-occlusion are both Lu/BCAM (BCAM: Basal Celle Adhesion Molecule) and ICAM-4 (ICAM: Intercellular Adhesion Molecule) expressed by the mature red blood cells and reticulocytes. In the case of sickle cell disease, these cells overexpress Lu/BCAM. Both Lu/BCAM and ICAM-4 promote adhesion of sickle erythrocytes to the endothelium through the interaction with the laminin 511/521 of the sub-endothelium extracellular matrix and endothelial integrin αVβ3, respectively. Lu blood group antigens and the basal cell adhesion molecule antigen (BCAM) are carried out by two glycoprotein isoforms of 85 and 78 kDa, respectively, and differ in the length of their C-terminal cytoplasmic domain. Therefore, Lu and BCAM share identical extracellular domains composed of one V-set and the four C2-set domains. Since these extracellular domains are identical and are those of interest in the present invention, these proteins are designated as Lu in the present invention. In the case of sickle cell disease, the glycoprotein Lu is the only receptor for laminin 511/521. Laminins are major protein components of the basal lamina and are heterotrimers formed by three subunits designed α, β and γ chains. These subunits form sixteen isoforms of laminins with laminin 511 and laminin 521 which both contain the α5 chain (Lnα5). Lnα5 consists of five globular domains LG1-LG5 at its c-terminus which can specifically interact with few integrins, α-dystroglycan, syndecan-4 and Lu. Lu seems to interact with a binding site found only in the α5 chain. Accession number in Uniprot for Laminin subunit alpha-5: 015230 (http://www.uniprot.org/uniprot/O15230). Laminin is a complex glycoprotein, consisting of three different polypeptide chains (alpha, beta, gamma). Laminin Alpha-5 is one of the alpha polypeptide chains found in laminin-10 (laminin-511), laminin-11 (laminin-521) and laminin-15 (laminin-523). Main references on Laminin alpha5 are:
"Tissue-specific expression of the human laminin alpha5-chain, and mapping of the gene to human chromosome 20q13.2-13.3 and to distal mouse chromosome 2 near the locus for the ragged (Ra) mutation." Durkin M.E., Loechel F., Mattei M.-G., Gilpin B.J., Albrechtsen R., Wewer U.M. FEBS Lett. 411:296-300(1997);
"Protein kinase A-dependent phosphorylation of Lutheran/basal cell adhesion molecule glycoprotein regulates cell adhesion to laminin alpha5." Gauthier E., Rahuel C., Wautier M.P., El Nemer W., Gane P., Wautier J.L., Cartron J.P., Colin Y., Le Van Kim C. J. Biol. Chem. 280:30055-30062(2005).

The only drug currently approved by the US Food and Drug Administration for the treatment of sickle cell disease is hydroxyurea, which inhibits the intracellular phosphorylation of Lu that occurs before interaction with Lnα5. This treatment significantly reduces the vaso-occlusive crisis in adults with sickle cell disease but is less effective in children. Furthermore, hydroxyurea treatment causes significant side effects such as bleeding or convulsions. It is therefore important to find other drug candidates to reduce the vaso-occlusion in patients with sickle cell disease. The inhibition of the interaction between Lu and Lnα5 is another strategy to fight the vaso-occlusion crisis. This strategy is also a major interest in the research of drugs again cancer since it has been found that the binding of Lu to Lnα5 promotes tumor cell migration (KIKKAWA et al., The Lutheran/Basal Cell Adhesion Molecule Promotes Tumor Cell Migration By Modulating Integrin-Mediated Cell Attachment To Lamin-511 Proterin. J. Biol. Chem.; 2013, 288, 30990-31001)*.* The search of protein-protein interaction inhibitors is notoriously difficult but is an important area in drug discovery because of the primary role of this type of interaction in many diseases. The search for inhibitors of the interaction Lu-Lnα5 has already been carried out in 2011 by KIKKAWA et al. (An Antibody To The Lutheran Glycoprotein (Lu) Recognizing The LU 4 Blood Type Variant Inhibits Cell Adhesion To Laminin A5, PLOS 1, 2011, 6, E233 29-E233-39)*.* They have found that an antibody directed against the second N-terminal domain of Lu could lead to an inhibition of the interaction by steric hindrance.

The present invention aims to provide new drugs and in particular new pharmaceutical compositions. These drugs or pharmaceutical compositions aim in particular to treat a genetic blood disorder and especially drepanocytosis.

One aim of the present invention is to provide a drug and a pharmaceutical composition inhibiting lutheran blood group glycoprotein-laminin 511/521 interaction.

It has been found that the molecules and the compositions according to the present invention solve the above described technical problems and are potent inhibitors of lutheran blood group glycoprotein-laminin 511/521 interaction.

A first screening has been made to identify inhibitors of Lutheran blood group glycoprotein-laminin 511/521 interaction by molecular modelling and simulation techniques. However, this modelling and simulation are not sufficient alone to support the therapeutic applications of the inhibitors. In the present invention, laboratory tests have been performed to support the therapeutic activity of the molecules as defined in the present invention. It is known that chances are very few that molecules that are predicted by docking techniques to bind a protein will actually bind this protein.

The present invention relates to molecules having the following chemical structure (I) or a salt thereof: 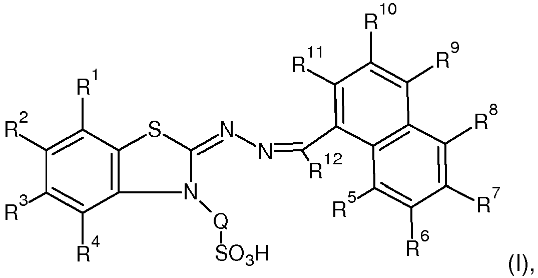 wherein R1 to R12 and Q are each independently an atom or a chemical group of atoms.

In one embodiment, said molecule has the chemical structure (II) or is a salt thereof: 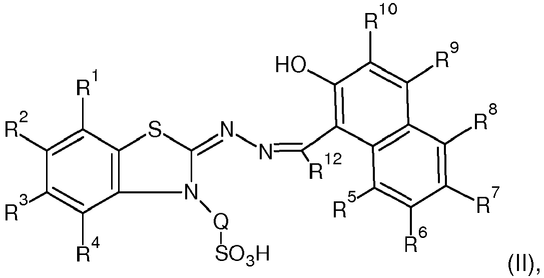 wherein R1 to R10, R12 and Q are each independently an atom or a chemical group of atoms.

In one embodiment, said molecule has the chemical structure (III) or is a salt thereof: 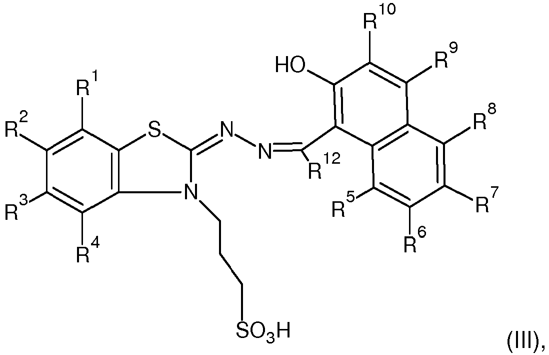 wherein R1 to R10 and R12 are each independently an atom or a chemical group of atoms.

In one embodiment, said molecule has the chemical structure (IV) or is a salt thereof: 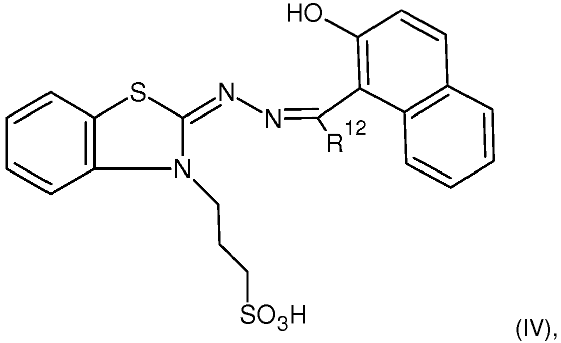 wherein R12 is an atom or a chemical group of atoms.

In one embodiment, said molecule has the chemical structure (V) or is a salt thereof:

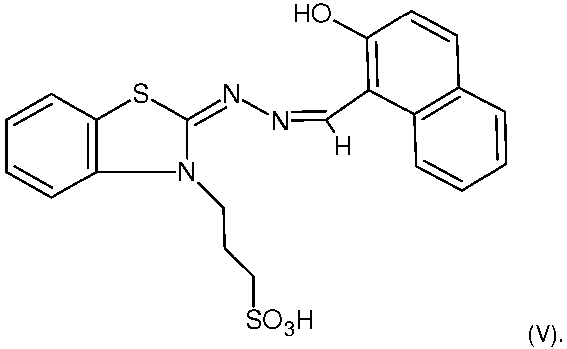

Molecules of formula (I) to (V) could also be represented by the following formula:

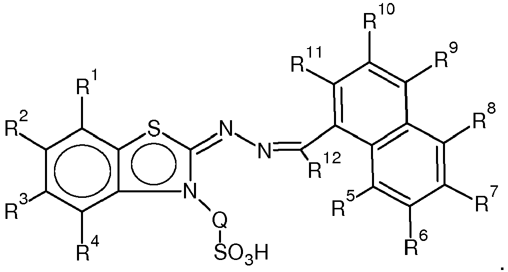

In one embodiment, R1 to R12, when designated in the structure, are each independently selected from the group consisting of hydrogen, halogen, cyano, a C₁-C₆ alkyl group, a C₃-C₇ cycloalkyl group, a C₁-C₄ alkoxy group, a C₁-C₄ haloalkyl group, - NRR', -NR-CO-R', and -CONRR' group wherein R and R' are each independently selected from hydrogen or a C₁-C₆ alkyl group, wherein Q is a substituted or unsubstituted C₁-C₆ chain.

In one embodiment, in any one of structures (I) to (IV), R1 to R10 and R12 when designated in the structure, are each independently selected from the group consisting of hydrogen, halogen, a C₁-C₆ alkyl group, a C₃-C₇ cycloalkyl group, a C₁-C₄ alkoxy group.

In one embodiment, in any one of structures (I) to (IV), R1 to R12, when designated in the structure, are each independently selected from the group consisting of hydrogen and a C₁-C₆ alkyl group.

In one embodiment, in any one of structures (I) to (IV), R1 to R10 and R12, when present in the structure, are each independently selected from the group consisting of hydrogen and a C₁-C₆ alkyl group.

In a specific embodiment, in any one of structures (I) to (IV), R1 to R12 when designated in the structure, represent independently a hydrogen atom, a hydroxyl or a methyl group.

In one embodiment, in any one of structures (I) to (IV), R1 to R10 and R12, represent independently a hydrogen atom or a methyl group.

"Substituted" means notably that at least one hydrogen atom of an atomic group is substituted by another atom or group of atoms, generally called substituent. For example one or more substituents are selected from a halogen, a halomethyl, a linear or branched C₁-C₆ alkyl group, a C₁-C₄ alkoxy group, an C₁-C₄ haloalkyl group, a C₁-C₄ hydroxyalkyl group, a C₃-C₇ cycloalkyl group, an aryl group, a heteroaryl group, a heterocycloalkyl group, a hydroxyl, a thioalkyl, a cyano, a haloalkoxy, a -C≡C-R, a solubilising group, - NRR', -NR-CO-R', -CONRR', SO₂R, -SO₂NRR', -NRSO₂R', -(CH₂)_{n'}-NRR', -O-(CH₂)_{n"}-NRR', -(CH₂)_{n'}-R", -O-(CH₂)_{n"}-R" group wherein R and R' are each independently selected from hydrogen, an alkyl group, or an aryl group or an heteroaryl group, R" is an heteroaryl or an heterocycloalkyl group, n' is 1 or 2 and n" is 2 or 3.

In one embodiment, in structure (I), R11 is a hydroxyl group.

The molecules according to the present invention include racemates, single enantiomers, non-racemic mixtures of enantiomers, pharmaceutically acceptable salts thereof and hydrates thereof.

Such configuration of a specific enantiomer concerns all embodiment and chemical structures of the molecules of the invention.

In one embodiment, a salt of any one of structures (I) to (V) shares the following structure:

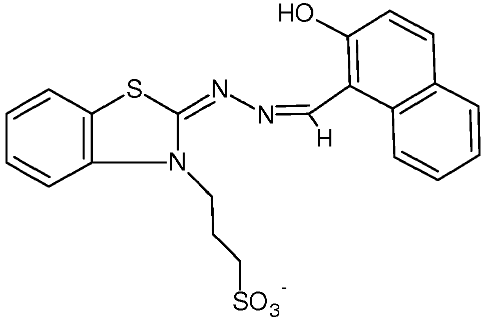

Salts are for example selected from the group consisting of salts obtained with bases, such as alkaline salts, in particular alkali metal hydroxides, for example obtained with sodium hydroxide, potassium hydroxide, etc., and acid addition salts, such as salts of hydrochloric acid, sulphuric acid, acetic acid, lactic acid, etc., and also and/or hydrates thereof.

In one embodiment the countercation is selected from Na+, K+, or a mixture thereof.

The invention relates in particular to a pharmaceutical composition comprising at least one molecule according to formula (I), (II), (III), (IV) or (V), or a salt thereof, and at least one excipient.

In particular, said pharmaceutical composition is for use in a method of therapeutic treatment of genetic blood disorder of a mammal.

In one embodiment, said pharmaceutical composition is for use in a method of therapeutic treatment of drepanocytosis of a mammal.

The invention also relates to a molecule having the chemical structure I), (II), (III), (IV) or (V), or a salt thereof, for use in a method of therapeutic treatment of a mammal:
Advantageously, molecules of the present invention limit Lu-Ln interaction.

It was unexpected and unpredictable that the molecules according to the present invention would limit Lu-Ln interaction.

In particular, said molecule is an inhibitor of Lutheran Blood group Glycoprotein - Lamenin 511/521 interaction.

In one embodiment, said molecule is for use in a method of therapeutic treatment of a genetic blood disorder of a mammal.

In one embodiment, said molecule is for use in a method of therapeutic treatment of drepanocytosis of a mammal.

The invention covers prodrugs of the molecules of the invention.

"Prodrug" means any molecule administered in an inactive or significantly less active form than after its bioactivation. Once administered, the prodrug is metabolised in vivo, in one or more steps, into a therapeutically active molecule (drug). A prodrug is usually not a therapeutically active molecule itself and will usually not elicit in vitro the biological response of the corresponding therapeutically active molecule after bioactivation.

In another aspect, the invention relates to a pharmaceutical composition comprising a molecule according to the present invention or one of its prodrugs, and also at least one pharmaceutically acceptable excipient. In another aspect, the invention relates to a process for preparing or manufacturing a pharmaceutical composition, said process comprising formulating at least one molecule according to the present invention with one or more excipients. In one embodiment, the invention relates to a process for preparing or manufacturing a pharmaceutical composition for use in a therapeutic treatment for a mammal.

This invention covers medical devices comprising a composition of the invention.

The molecules as defined in the present invention are notably for use in a method of therapeutic treatment of a mammal, especially a human being.

In particular, the molecules and compositions of the present invention are for use in the method of therapeutic treatment of a genetic blood disorder of a mammal, especially a human being.

More particularly, the molecules and compositions according to the present invention are for use in the method of therapeutic treatment of drepanocytosis of a mammal, especially a human being.

Also, the present invention relates to a method of therapeutic treatment including genetic blood disorder treatment and drepanocytosis treatment of a mammal, especially a human being.

Advantageously, the molecule and compositions according to the present invention are administered in an efficient amount to a human being in need of said treatment.

"Mammal" refers to humans or any animals including, but not limited to Primate (including humans, apes and monkeys), Arteriodactyla (including horses, goats, cows, sheep, pigs), Rodenta (including mice, rats, rabbits, and hamsters), and Carnivora (including cats, and dogs). Among birds, the mammals include, but are not limited to, turkeys, chickens and other members of the same order. In a preferred embodiment, the mammals are humans as the intended use of the invention formulation is human pharmaceutical applications. In addition, the invention formulation can also be suitable for veterinary applications without further manipulations that changes the excipients or excipient ratios that are present.

The amount of the active ingredient or the pharmaceutical composition which constitutes a "therapeutically effective amount" will vary depending on the active ingredient, the pharmaceutical composition, the disease or condition and its severity, other conditions affecting the health of the mammal to be treated, the manner of administration, and the age of the mammal to be treated, but can be determined routinely by one of ordinary skill in the art having regard to his own knowledge and to this disclosure.

"Pharmaceutically acceptable excipient" includes without limitation any solvent, adjuvant, bioavailability enhancer, carrier, glidant, sweetening agent, diluent, preservative, dye/colorant, flavor enhancer, solubilizer (including surfactants), wetting agent, dispersing agent, suspending agent, stabilizer, isotonic agent, buffer or emulsifier which has been approved by the a Administration for drug, medicine or Health regulation, as being acceptable for use in humans or mammals. "Pharmaceutically acceptable salt" includes both acid and base addition salts.

In one embodiment, the pharmaceutical composition comprises water.

Pharmaceutical composition can be adapted for oral administration, and can be formulated using pharmaceutically acceptable carriers well known in the art in suitable dosages. Such carriers enable the pharmaceutical compositions to be formulated as tablets, pills, dragees, capsules, liquids, gels, syrups, slurries, suspensions, and the like, for ingestion by the patient. In addition to the active ingredients, these pharmaceutical compositions may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, Pa.).

Accordingly, the present invention also relates to an oral pharmaceutical composition.

In the figures:
Figure 1: Adhesion of RBCs from sickle cell disease patients (N = 3) to laminin 521. Results are presented as a percentage of adhesion, 100% of adhesion being the adhesion level observed for RBCs incubated with buffer supplemented with DMSO alone.
Figure 2: Adhesion of RBCs from sickle cell disease patients to laminin 521 in the presence of increasing concentrations of Molecule 2 and Molecule 3. Results are presented as a percentage of adhesion, 100% of adhesion being the adhesion level observed for RBCs incubated without the molecules.

### Examples

### Example 1 - Adhesion test on Ln

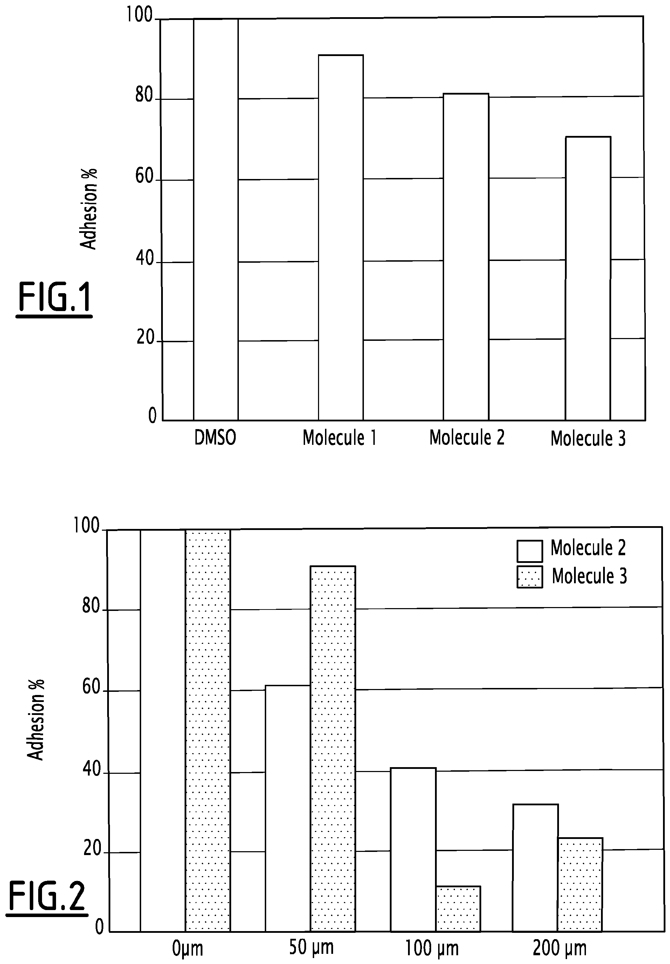

Red blood cell adhesion to laminin 521 was measured under flow conditions using Vena8 Endothelial+™ biochips (internal channel dimensions: length 20 mm, width 0.8 mm, height 0.12 mm) and ExiGo™ Nanopumps (Cellix Ltd, Dublin, Ireland). Three different solutions were prepared with either Molecule 1, Molecule 2 or Molecule 3 (0.1 mM) by solubilizing Molecule 1, 2 or 3 using DMSO. Recombinant human laminin 521 (BioLamina) at 5 ng/µl was immobilized on the internal surface of the biochips at 4°C overnight. RBCs from sickle cell disease patients were suspended at 5x10⁷ cells/ml in Hanks balanced salt solution, without calcium chloride and magnesium sulfate (Sigma-Aldrich) supplemented with 0.4% BSA, and incubated with the solution comprising either Molecule 1, Molecule 2 or Molecule 3 at 0.1 mM or DMSO, for 30 min at room temperature (20°C) and perfused through the biochip channels for 10 min at a shear stress of 0.5 dyn/cm² followed by a washout at the same shear stress with the buffer alone for 30 min. Five minutes washouts with the Hanks/0.4% BSA buffer were performed at 1, 2, 3, 4 and 5 dyn/cm². After each wash, adherent RBCs were counted in 7 representative areas along the centerline of each channel using the AxioObserver Z1 microscope and ZEN analysis software (Carl Zeiss, Le Pecq, France) and the mean number of adherent RBCs was determined for each condition and each washout step (Figure 1). Images of the same 7 areas were obtained throughout each experiment. Three adhesion assays were performed with 3 different blood samples. RBC adhesion to laminin was inhibited when the RBC suspension was incubated with Molecule 2 or 3, but not Molecule 1.

Molecule 1 presents the following chemical structure:

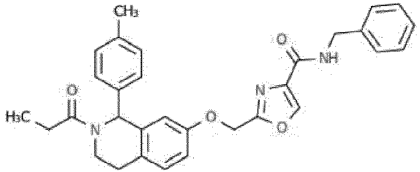

Molecule 2 presents the following chemical structure:

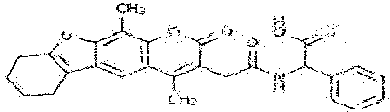

Molecule 3 presents the following chemical structure:

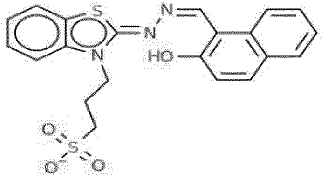

### Example 2 - Control of hemolysis

At the end of the 30 min of incubation with the molecules, the number of RBC for the control of hemolysis was measure by using CASY® Cell Counter and Analyzer.

For hemolysis assays, RBCs were suspended at 6x10⁶ cells/ml in the Hanks/0.4% BSA buffer and the concentration measured accurately using the CASY® Cell Counter and Analyzer. RBCs were then incubated with the solution comprising either Molecule 1, Molecule 2 or Molecule 3 at 0.1 mM or DMSO, for 30 min at room temperature. After this incubation time cell concentration was measured again using CASY® Cell Counter and Analyzer (Table 1). If hemolysis occurs during this incubation time it will be reflected by a decrease in the RBC concentration.

**Table 1**

| Incubation condition | RBCs/ml (x10⁶) |
|---|---|
| Buffer alone | 6.125 |
| Buffer + DMSO | 6.04 |
| Molecule 1 | 6.04 |
| Molecule 2 | 5.55 |
| Molecule 3 | 6.02 |

There was no decrease of RBC concentration in the presence of Molecule 1 or 3 compared to vehicle (DMSO) alone. A slight decrease of the concentration (< 10%) was observed in the presence of Molecule 2 suggesting a potential small hemolytic effect for this molecule.

### Example 3 - Dose effect

To address the dose effect of the molecules, adhesion assays were performed in the same conditions as in Example 1, using the following concentrations for Molecule 1, 2 or 3:
50, 100 and 200 µM.

The experimental results are shown on Figure 2.

Increasing concentrations of Molecules 2 and 3 showed increasing inhibition of RBC adhesion to laminin. This inhibitory dose-dependent effect supports the specificity of both molecules to interact with Lu while inhibiting its interaction with laminin.

Accordingly, molecules of the invention inhibiting Lu-Ln interaction should have a therapeutic activity in diseases involving Lu-Ln interaction, such as in genetic blood disorder involving Lu-Ln interaction, in particular drepanocystosis.

## Claims

1. A pharmaceutical composition for a mammal comprising a compound having the chemical structure (I) or a salt thereof: 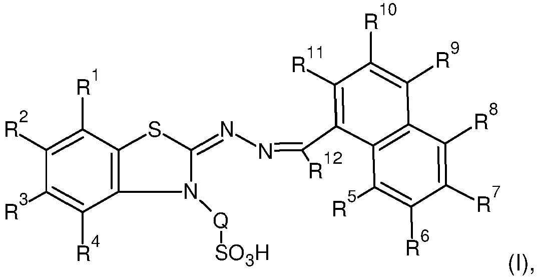
wherein R1 to R10 and R12 and Q are each independently selected from the group consisting of hydrogen, halogen, cyano, a C₁-C₆ alkyl group, a C₃-C₇ cycloalkyl group, a C₁-C₄ alkoxy group, a C₁-C₄ haloalkyl group, -NRR', -NR-CO-R', and -CONRR' group wherein R and R' are each independently selected from hydrogen or a C₁-C₆ alkyl group, wherein Q is a substituted or unsubstituted C₁-C₆ chain,
wherein R11 is a hydroxyl group,
and at least one excipient.

2. The pharmaceutical composition according to claim 1, wherein said compound has the chemical structure (II) or is a salt thereof: 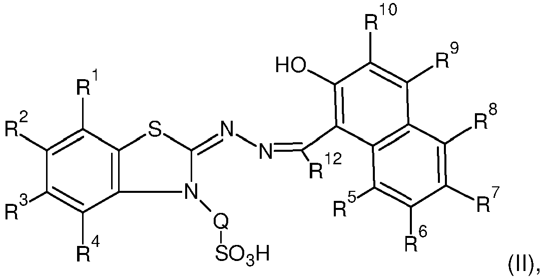 wherein R1 to R10 and R12 are each independently selected from the group consisting of hydrogen, halogen, a C₁-C₆ alkyl group, a C₃-C₇ cycloalkyl group, and a C₁-C4 alkoxy group.

3. The pharmaceutical composition according to claim 1, wherein said compound has the chemical structure (III) or is a salt thereof: 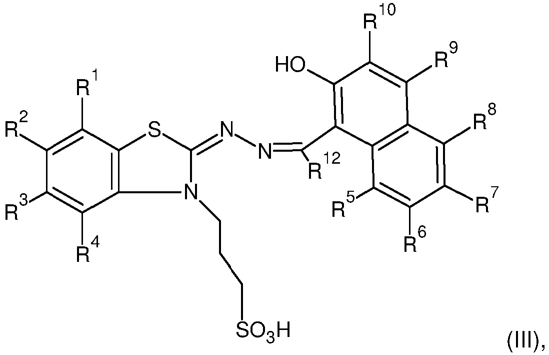 wherein R1 to R10 and R12 are each independently selected from the group consisting of hydrogen, halogen, a C₁-C₆ alkyl group, a C₃-C₇ cycloalkyl group, and a C₁-C4 alkoxy group.

4. The pharmaceutical composition according to claim 1, wherein said compound has the chemical structure (IV) or is a salt thereof: 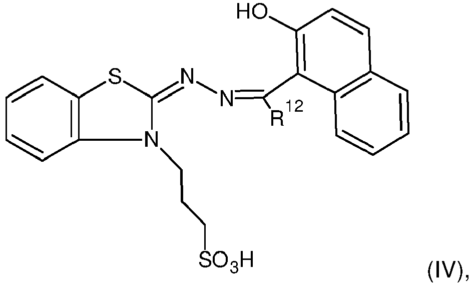 wherein R12 is selected from the group consisting of hydrogen, halogen, a C₁-C₆ alkyl group, a C₃-C₇ cycloalkyl group, and a C₁-C₄ alkoxy group.

5. The pharmaceutical composition according to claim 1, wherein said compound has the chemical structure (V) or is a salt thereof:

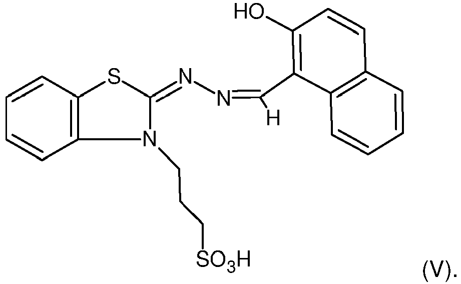

6. The pharmaceutical composition according to claim 1, wherein said pharmaceutical composition is for use in a method of therapeutic treatment of genetic blood disorder of a mammal.

7. The pharmaceutical composition according to claim 1, wherein said pharmaceutical composition is for use in a method of therapeutic treatment of drepanocytosis of a mammal.

8. A compound having the chemical structure (I) or a salt thereof for use in a method of therapeutic treatment of a mammal: 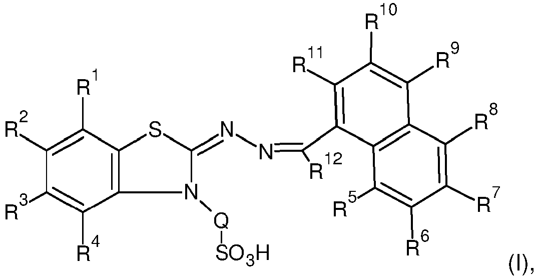 wherein R1 to R10 and R12 and Q are each independently selected from the group consisting of hydrogen, halogen, cyano, a C₁-C₆ alkyl group, a C₃-C₇ cycloalkyl group, a C₁-C₄ alkoxy group, a C₁-C₄ haloalkyl group, -NRR', -NR-CO-R', and -CONRR' group wherein R and R' are each independently selected from hydrogen or a C₁-C₆ alkyl group, wherein Q is a substituted or unsubstituted C₁-C₆ chain, and
wherein R11 is a hydroxyl group.

9. The compound for use according to claim 8, wherein said compound has the chemical structure (II) or is a salt thereof: 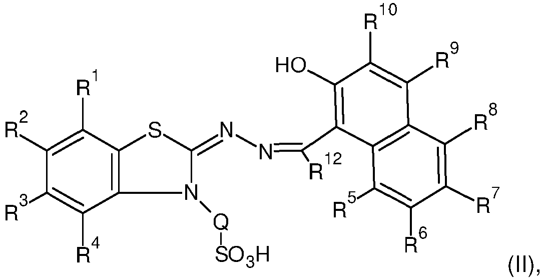 wherein R1 to R10, R12 and Q are each independently selected from the group consisting of hydrogen, halogen, a C₁-C₆ alkyl group, a C₃-C₇ cycloalkyl group, and a C₁-C4 alkoxy group.

10. The compound for use according to claim 8, wherein said compound has the chemical structure (III) or is a salt thereof: 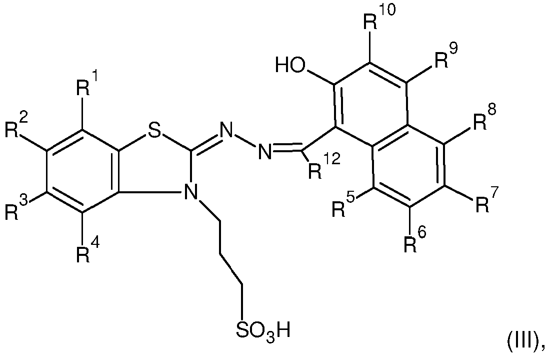 wherein R1 to R10 and R12 are each independently selected from the group consisting of hydrogen, halogen, a C₁-C₆ alkyl group, a C₃-C₇ cycloalkyl group, and a C₁-C4 alkoxy group.

11. The compound for use according to claim 8, wherein said compound has the chemical structure (IV) or is a salt thereof: 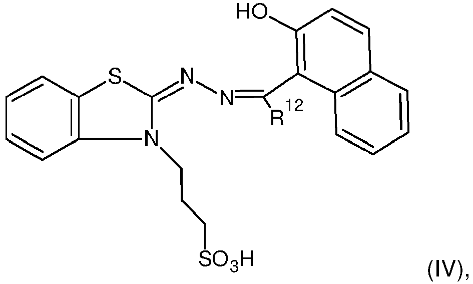 wherein R12 is selected from the group consisting of hydrogen, halogen, a C₁-C₆ alkyl group, a C₃-C₇ cycloalkyl group, and a C₁-C₄ alkoxy group.

12. The compound for use according to claim 8, wherein said compound has the chemical structure (V) or is a salt thereof:

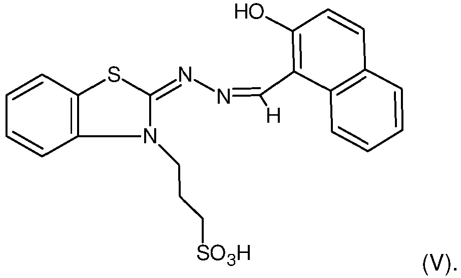

13. The compound for use according to any one of claims 8 to 12, wherein said compound is an inhibitor of Lutheran Blood group Glycoprotein - Lamenin 511/521 interaction.

14. The compound according to any one of claims 8 to 12 for use in a method of therapeutic treatment of a genetic blood disorder of a mammal.

15. The compound according to any one of claims 8 to 12 for use in a method of therapeutic treatment of drepanocytosis of a mammal.

## Patentansprüche

1. Pharmazeutische Zusammensetzung für ein Säugetier, aufweisend eine Verbindung mit der chemischen Struktur (I) oder ein Salz dieser: 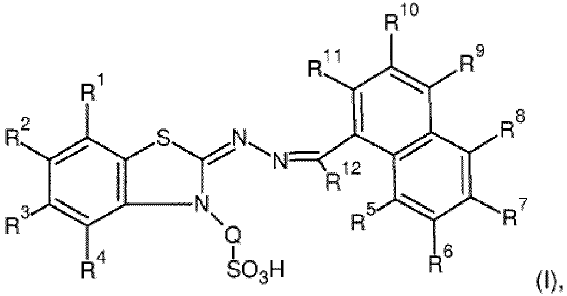
wobei R1 bis R10 und R12 und Q jeweils unabhängig voneinander ausgewählt sind aus der Gruppe bestehend aus Wasserstoff, Halogen, Cyano, einer C₁-C₆-Alkylgruppe, einer C₃-C₇-Cycloalkylgruppe, einer C₁-C₄-Alkoxygruppe, einer C₁-C₄-Halogenalkylgruppe, -NRR', -NR-CO-R' und - CONRR' Gruppe,
wobei R und R' jeweils unabhängig voneinander ausgewählt sind aus Wasserstoff oder einer C₁-C₆-Alkylgruppe,
wobei Q eine substituierte oder unsubstituierte C₁-C₆-Kette ist,
wobei R11 eine Hydroxylgruppe ist,
und mindestens einen Hilfsstoff.

2. Pharmazeutische Zusammensetzung nach Anspruch 1, wobei die besagte Verbindung die chemische Struktur (II) aufweist oder ein Salz dieser ist: 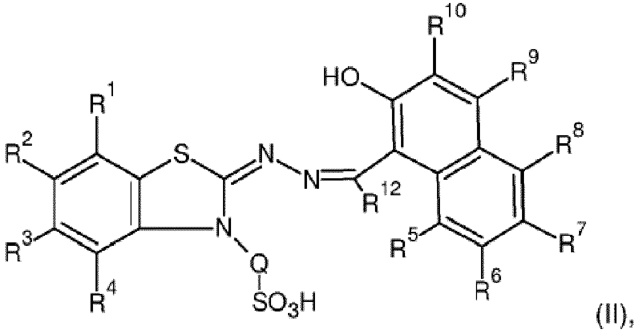 wobei R1 bis R10 und R12 jeweils unabhängig voneinander ausgewählt sind aus der Gruppe bestehend aus Wasserstoff, Halogen, einer C₁-C₆-Alkylgruppe, einer C₃-C₇-Cycloalkylgruppe und einer C₁-C₄-Alkoxygruppe.

3. Pharmazeutische Zusammensetzung nach Anspruch 1, wobei die besagte Verbindung die chemische Struktur (III) aufweist oder ein Salz dieser ist: 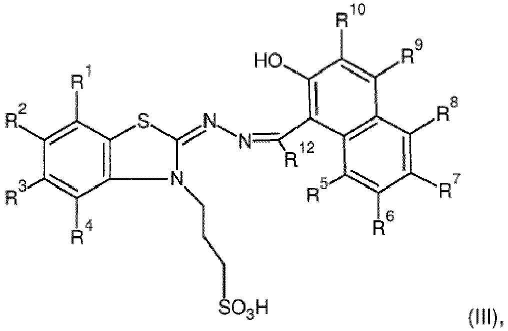 wobei R1 bis R10 und R12 jeweils unabhängig voneinander ausgewählt sind aus der Gruppe bestehend aus Wasserstoff, Halogen, einer C₁-C₆-Alkylgruppe, einer C₃-C₇-Cycloalkylgruppe und einer C₁-C₄-Alkoxygruppe.

4. Pharmazeutische Zusammensetzung nach Anspruch 1, wobei die besagte Verbindung die chemische Struktur (IV) aufweist oder ein Salz dieser ist: 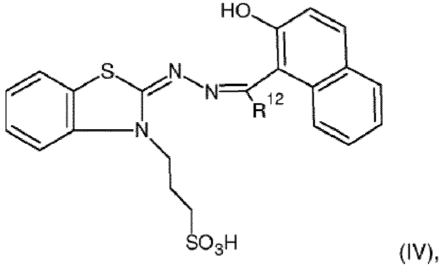 wobei R12 ausgewählt ist aus der Gruppe bestehend aus Wasserstoff, Halogen, einer C₁-C₆-Alkylgruppe, einer C₃-C₇-Cycloalkylgruppe und einer C₁-C₄-Alkoxygruppe.

5. Pharmazeutische Zusammensetzung nach Anspruch 1, wobei die besagte Verbindung die chemische Struktur (V) aufweist oder ein Salz dieser ist:

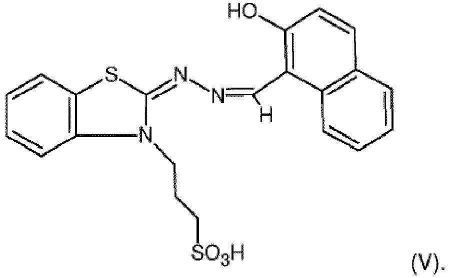

6. Pharmazeutische Zusammensetzung nach Anspruch 1, wobei die besagte pharmazeutische Zusammensetzung zur Verwendung in einem Verfahren zur therapeutischen Behandlung einer genetischen Blutstörung eines Säugetiers bestimmt ist.

7. Pharmazeutische Zusammensetzung nach Anspruch 1, wobei die besagte pharmazeutische Zusammensetzung zur Verwendung in einem Verfahren zur therapeutischen Behandlung von Drepanozytose eines Säugetiers bestimmt ist.

8. Eine Verbindung mit der chemischen Struktur (I) oder ein Salz dieser zur Verwendung in einem Verfahren zur therapeutischen Behandlung eines Säugetiers: 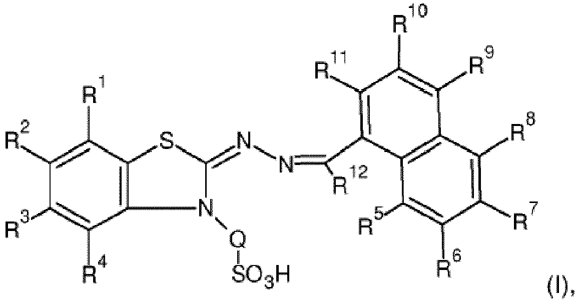
wobei R1 bis R10 und R12 und Q jeweils unabhängig voneinander ausgewählt sind aus der Gruppe bestehend aus Wasserstoff, Halogen, Cyano, einer C₁-C₆-Alkylgruppe, einer C₃-C₇-Cycloalkylgruppe, einer C₁-C₄-Alkoxygruppe, einer C₁-C₄-Halogenalkylgruppe, -NRR', -NR-CO-R' und - CONRR' Gruppe,
wobei R und R' jeweils unabhängig voneinander ausgewählt sind aus Wasserstoff oder einer C₁-C₆-Alkylgruppe,
wobei Q eine substituierte oder unsubstituierte C₁-C₆-Kette ist, und
wobei R11 eine Hydroxylgruppe ist.

9. Verbindung zur Verwendung nach Anspruch 8, wobei die besagte Verbindung die chemische Struktur (II) aufweist oder ein Salz dieser ist: 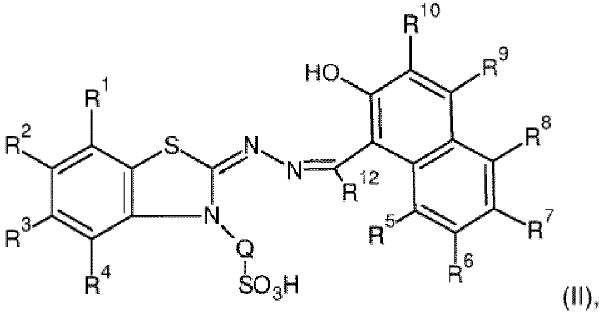 wobei R1 bis R10, R12 und Q jeweils unabhängig voneinander ausgewählt sind aus der Gruppe bestehend aus Wasserstoff, Halogen, einer C₁-C₆-Alkylgruppe, einer C₃-C₇-Cycloalkylgruppe und einer C₁-C₄-Alkoxygruppe.

10. Verbindung zur Verwendung nach Anspruch 8, wobei die besagte Verbindung die chemische Struktur (III) aufweist oder ein Salz dieser ist: 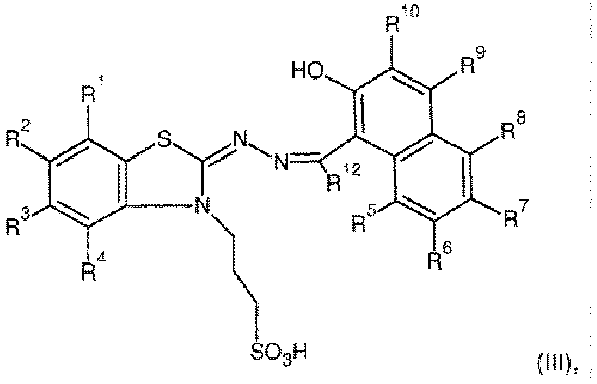 wobei R1 bis R10 und R12 jeweils unabhängig voneinander ausgewählt sind aus der Gruppe bestehend aus Wasserstoff, Halogen, einer C₁-C₆-Alkylgruppe, einer C₃-C₇-Cycloalkylgruppe und einer C₁-C₄-Alkoxygruppe.

11. Verbindung zur Verwendung nach Anspruch 8, wobei die besagte Verbindung die chemische Struktur (IV) aufweist oder ein Salz davon ist: 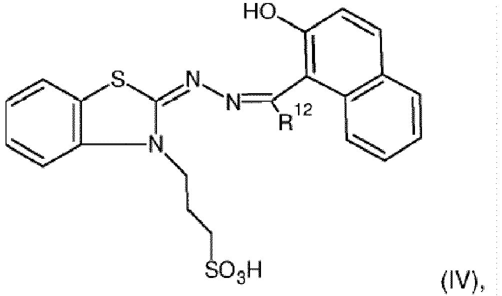 wobei R12 ausgewählt ist aus der Gruppe bestehend aus Wasserstoff, Halogen, einer C₁-C₆-Alkylgruppe, einer C₃-C₇-Cycloalkylgruppe und einer C₁-C₄-Alkoxygruppe.

12. Verbindung zur Verwendung nach Anspruch 8, wobei die besagte Verbindung die chemische Struktur (V) aufweist oder ein Salz dieser ist:

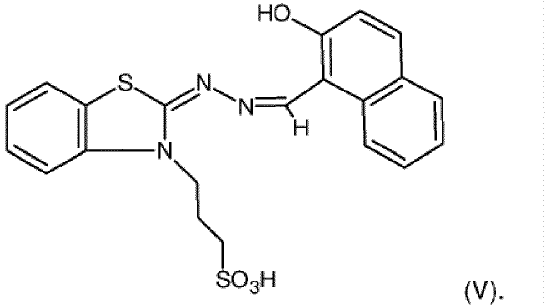

13. Verbindung zur Verwendung nach irgendeinem der Ansprüche 8 bis 12, wobei die besagte Verbindung ein Inhibitor der Lutheran-Blutgruppe Glycoprotein - Lamenin 511/521 Wechselwirkung ist.

14. Verbindung nach irgendeinem der Ansprüche 8 bis 12 zur Verwendung in einem Verfahren zur therapeutischen Behandlung einer genetischen Blutstörung eines Säugetiers.

15. Verbindung nach irgendeinem der Ansprüche 8 bis 12 zur Verwendung in einem Verfahren zur therapeutischen Behandlung von Drepanozytose eines Säugetiers.

## Revendications

1. Composition pharmaceutique pour un mammifère comprenant un composé ayant la structure chimique (I) ou un sel de celui-ci : 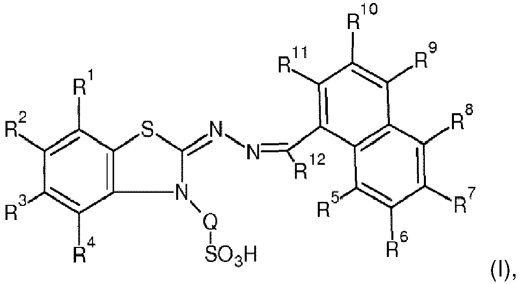
dans laquelle R1 à R10 et R12 et Q sont chacun indépendamment choisis dans le groupe constitué d'hydrogène, halogène, cyano, un groupe alkyle en C₁-C₆, un groupe cycloalkyle en C₃-C7, un groupe alcoxy en C₁-C₄, un groupe halogénoalkyle en C₁-C₄, un groupe -NRR', -NR-CO-R' et -CONRR', où R et R' sont chacun indépendamment choisis parmi hydrogène ou un groupe alkyle en C₁-C₆, dans laquelle Q est une chaîne en C₁-C₆ substituée ou non substituée,
dans laquelle R11 est un groupe hydroxyle,
et au moins un excipient.

2. Composition pharmaceutique selon la revendication 1, dans laquelle ledit composé a la structure chimique (II) ou est un sel de celle-ci : 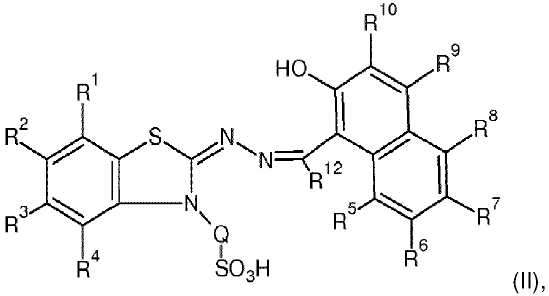 dans laquelle R1 à R10 et R12 sont chacun indépendamment choisis dans le groupe constitué d'hydrogène, halogène, un groupe alkyle en C₁-C₆, un groupe cycloalkyle en C₃-C₇ et un groupe alcoxy en C₁-C₄.

3. Composition pharmaceutique selon la revendication 1, dans laquelle ledit composé a la structure chimique (III) ou est un sel de celle-ci : 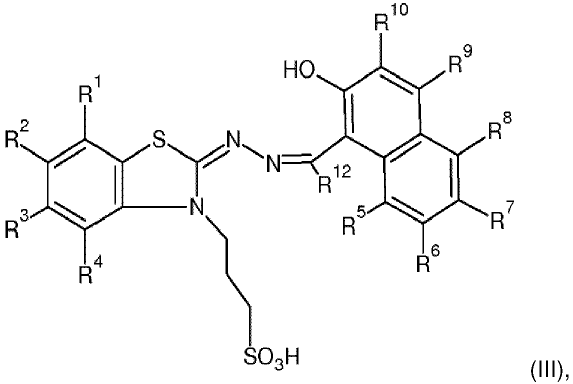 dans laquelle R1 à R10 et R12 sont chacun indépendamment choisis dans le groupe constitué d'hydrogène, halogène, un groupe alkyle en C₁-C₆, un groupe cycloalkyle en C₃-C₇ et un groupe alcoxy en C₁-C₄.

4. Composition pharmaceutique selon la revendication 1, dans laquelle ledit composé a la structure chimique (IV) ou est un sel de celle-ci : 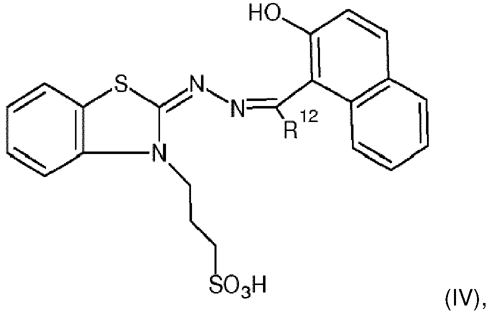 dans laquelle R12 est choisi dans le groupe constitué d'hydrogène, halogène, un groupe alkyle en C₁-C₆, un groupe cycloalkyle en C₃-C₇ et un groupe alcoxy en C₁-C₄.

5. Composition pharmaceutique selon la revendication 1, dans laquelle ledit composé a la structure chimique (V) ou est un sel de celle-ci :

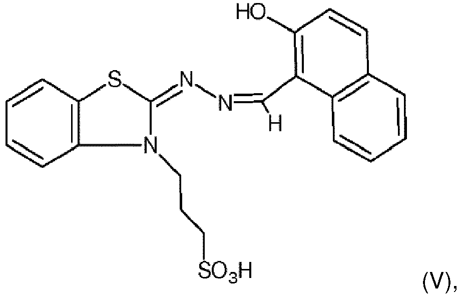

6. Composition pharmaceutique selon la revendication 1, où ladite composition pharmaceutique est pour utilisation dans un procédé de traitement thérapeutique d'un trouble sanguin génétique chez un mammifère.

7. Composition pharmaceutique selon la revendication 1, où ladite composition pharmaceutique est pour utilisation dans un procédé de traitement thérapeutique de la drépanocytose chez un mammifère.

8. Composé ayant la structure chimique (I) ou sel de celui-ci pour utilisation dans un procédé de traitement thérapeutique d'un mammifère : 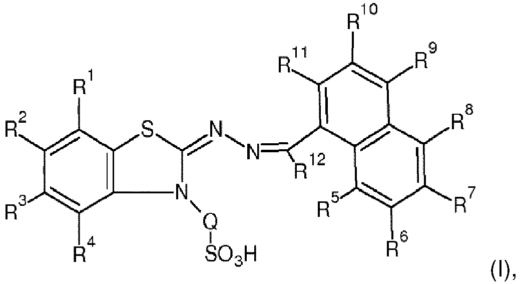 dans laquelle R1 à R10 et R12 et Q sont chacun indépendamment choisis dans le groupe constitué d'hydrogène, halogène, cyano, un groupe alkyle en C₁-C₆, un groupe cycloalkyle en C₃-C₇, un groupe alcoxy en C₁-C₄, un groupe halogénoalkyle en C₁-C₄, un groupe -NRR', -NR-CO-R' et -CONRR' où R et R' sont chacun indépendamment choisis parmi hydrogène ou un groupe alkyle en C₁-C₆, dans laquelle Q est une chaîne en C₁-C₆ substituée ou non substituée, et
dans laquelle R11 est un groupe hydroxyle.

9. Composé pour utilisation selon la revendication 8, où ledit composé a la structure chimique (II) ou est un sel de celle-ci : 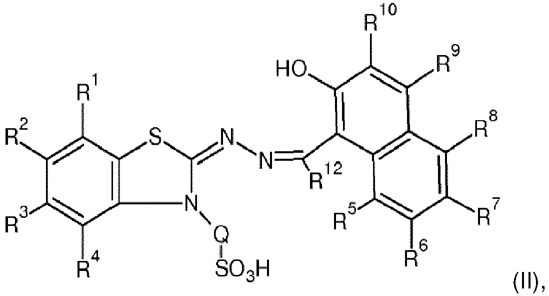 dans laquelle R1 à R10, R12 et Q sont chacun indépendamment choisis dans le groupe constitué d'hydrogène, halogène, un groupe alkyle en C₁-C₆, un groupe cycloalkyle en C₃-C₇ et un groupe alcoxy en C₁-C₄.

10. Composé pour utilisation selon la revendication 8, où ledit composé a la structure chimique (III) ou est un sel de celle-ci : 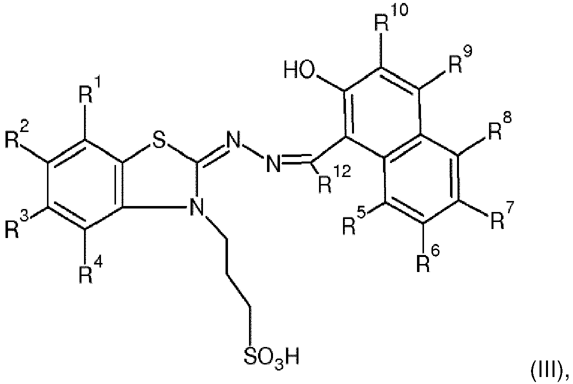 dans laquelle R1 à R10 et R12 sont chacun indépendamment choisis dans le groupe constitué d'hydrogène, halogène, un groupe alkyle en C₁-C₆, un groupe cycloalkyle en C₃-C₇ et un groupe alcoxy en C₁-C₄.

11. Composé pour utilisation selon la revendication 8, où ledit composé a la structure chimique (IV) ou est un sel de celle-ci : 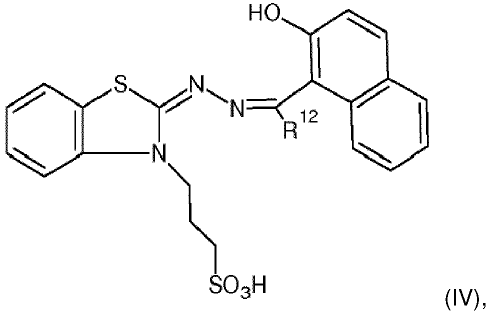 dans laquelle R12 est choisi dans le groupe constitué d'hydrogène, halogène, un groupe alkyle en C₁-C₆, un groupe cycloalkyle en C₃-C₇ et un groupe alcoxy en C₁-C₄.

12. Composé pour utilisation selon la revendication 8, où ledit composé a la structure chimique (V) ou est un sel de celle-ci :

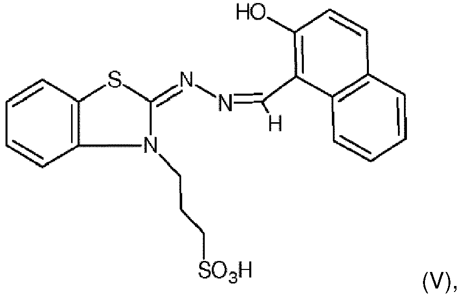

13. Composé pour utilisation selon l'une quelconque des revendications 8 à 12, où ledit composé est an inhibiteur de l'interaction glycoprotéine de groupe sanguin Lutheran - laménine 511/521.

14. Composé selon l'une quelconque des revendications 8 à 12 pour utilisation dans un procédé de traitement thérapeutique d'un trouble sanguin génétique chez un mammifère.

15. Composé selon l'une quelconque des revendications 8 à 12 pour utilisation dans un procédé de traitement thérapeutique de la drépanocytose chez un mammifère.
